# EUROPÄISCHE PATENTSCHRIFT

(11) **EP 1 833 810 B1**
(45) Veröffentlichungstag und Bekanntmachung des Hinweises auf die Patenterteilung: **30.08.2017**
(21) Anmeldenummer: 05808401.3
(22) Anmeldetag: 24.11.2005
(51) Int. Cl.: C07D 295/185

(54) **KRISTALLINE MODIFIKATIONEN VON N-ALPHA-(2,4,6-TRIISOPROPYLPHENYLSULFONYL)-3-HYDROXYAMIDINO-(L)-PHENYLALANIN-4-ETHOXYCARBONYLPIPERAZID UND/ODER SALZEN DAVON**
CRYSTALLINE MODIFICATIONS OF N-ALPHA-(2,4,6-TRIISOPROPYLPHENYLSULFONYL)-3-HYDROXYAMIDINO-(L)-PHENYLALANINE-4-ETHOXYCARBONYLPIPERAZIDE AND/OR SALTS THEREOF
MODIFICATIONS CRISTALLINES DU COMPOSÉ N-ALPHA-(2,4,6-TRIISOPROPYLPHENYLSULFONYL)-3-HYDROXYAMIDINO-(L)-PHENYLALANIN-4-ETHOXYCARBONYLPIPERAZIDE ET/OU DES SELS DE CE COMPOSÉ

(30) Priorität: 26.11.2004 DE 102004057195
(43) Veröffentlichungstag der Anmeldung: 19.09.2007
(73) Patentinhaber: Wilex AG, 81675 München (DE)
(72) Erfinder: GRUNENBERG, Alfons, 41539 Dormagen (DE); LENZ, Jana, 70771 Leinfelden-Echterdingen (DE)
(74) Vertreter: Weickmann & Weickmann PartmbB
(86) Internationale Anmeldenummer: PCT/EP2005/012589
(87) Internationale Veröffentlichungsnummer: WO 2006/056448

(56) Entgegenhaltungen:
- WO-A-03/072559
- WO-A-2004/011449
- WO-A-2004/067522
- WO-A-2004/103984
- US-A1- 2004 110 831
- US-A1- 2005 245 757
- BAVIN M: "POLYMORPHISM IN PROCESS DEVELOPMENT", CHEMISTRY & INDUSTRY, SOCIETY OF CHEMICAL INDUSTRY. LONDON, GB, vol. 21, 21 August 1989 (1989-08-21), pages 527-529, XP001180136, ISSN: 0009-3068
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: 10.1007/3-540-69178-2_5

## Beschreibung

Die vorliegende Erfindung betrifft neue kristalline Modifikationen von N-α-(2,4,6-Triisopropylphenylsulfonyl)-3-hydroxyamidino-(L)-phenylalanin-4-ethoxycarbonylpiperazid und/oder Sulfat-Salzen davon, welche als pharmazeutische Mittel verwendbar sind, sowie diese neuen kristallinen Modifikationen enthaltende pharmazeutische Zusammensetzungen und pharmazeutische Verwendungen.

Die neuen kristallinen Modifikationen der vorliegenden Erfindung, welche von einer Verbindung ausgehen, die unter dem chemischen Namen N-α-(2,4,6-Triisopropylphenylsulfonyl)-3-hydroxyamidino-(L)-phenylalanin-4-ethoxycarbonyl-piperazid bekannt ist, sind wirksame Inhibitoren der Serinprotease Urokinase und daher besonders geeignet zur Behandlung von Urokinaseassoziierten Erkrankungen, wie z.B. Tumore und Metastasierung, insbesondere zur oralen Verwendung. Die freie Basenform wird als WX-671 bezeichnet.

Der Plasminogenaktivator vom Urokinase-Typ (uPA) spielt eine Schlüsselrolle bei der Tumorinvasion und Metastasenbildung (Schmitt et al., J. Obst. Gyn. 21 (1995), 151-165). uPA wird in den verschiedensten Arten von Tumorzellen exprimiert (Kwaan, Cancer Metastasis Rev. 11 (1992), 291-311) und bindet an den Tumor-assoziierten uPA-Rezeptor (uPAR), wo die Aktivierung von Plasminogen zu Plasmin stattfindet. Plasmin ist in der Lage, verschiedene Komponenten der extrazellulären Matrix (ECM), wie Fibronectin, Laminin und Kollagen Typ IV, abzubauen. Es aktiviert auch einige andere ECM-abbauende Enzyme, insbesondere Matrix-Metalloproteinasen. Hohe Mengen an Tumor assoziierten uPA korrelieren mit einem höheren Metastasierungsrisiko für Krebspatienten (Harbeck et al., Cancer Research 62 (2002), 4617-4622). Eine Hemmung der proteolytischen Aktivität von uPA ist daher ein guter Ansatzpunkt für eine anti-metastatische Therapie.

Einige aktive und selektive Urokinase-Inhibitoren sind schon beschrieben. So werden uPA-Inhibitoren des Benzamidin-Typs in EP 1 098 651, uPA-Inhibitoren des Arylguanidin-Typs in WO 01/96286 und WO 02/14349 offenbart. Ein gemeinsames Merkmal dieser synthetischen Inhibitoren ist ein basischer Rest bestehend aus einer Amidino- oder/und Guanidino-Gruppe.

Die internationale Patentanmeldung WO 03/072559 offenbart WX-671 als Zwischenprodukt, bei der Synthese des Urokinase-Hemmstoffs N-α-(2,4,6-Triisopropylphenylsulfonyl)-3-amidino-(L)-phenylalanin-4-ethoxycarbonylpiperazid. WO 03/072559 offenbart auch ein Verfahren zur Herstellung der Verbindung WX-671 als freie Base oder in Form ihrer mit Säuren gebildeten Salze. Spezielle Salze, insbesondere die Hydrogensulfat- und die Sulfatform, sind nicht erwähnt. Ebenfalls nicht beschrieben sind Verfahren zur Kristallisierung. Bei den beschriebenen Verfahren wird die freie Base WX-671 als amorphes Produkt erhalten, welches thermisch leicht instabil und hygroskopisch ist und welches ungeeignete Filtrations- und Trocknungseigenschaften aufweist. Aus diesem Grund ist es zur Herstellung in großem Maßstab nicht geeignet und muss vor allem vor Wärme und Feuchtigkeit geschützt werden.

WO 2004/011449 offenbart ebenfalls Verfahren zur Herstellung von Phenylalanin-Derivaten, welche auch als Salze, z.B. als Salze von Mineralsäuren oder als Salze von organischen Säuren, vorliegen können. Eine der hergestellten Verbindungen ist WX-671. Auch hier ist kein Hinweis darauf gegeben, dass die offenbarten Verbindungen auch in einer stabilen kristallinen Form erhalten werden können.

PCT/EP2004/005682 offenbart Hydroxyamin- und Hydroxyguanidin-Verbindungen als Urokinase-Hemmstoffe. Die offenbarten Arzneimittel umfassen als Wirkstoff u.a. WX-671 und die Wirkstoffe können als Salze, z.B. als Hydrochlorid oder Hydrogensulfat oder als Salze von organischen Säuren, vorliegen. Für die dort beanspruchten Arzneimittel wurde eine bessere Bioverfügbarkeit bei oraler Verabreichung geltend gemacht. Auch diese Veröffentlichung offenbart jedoch keine Sulfatsalzverbindung der genannten Urokinase-Hemmstoffe.

Die Aufgabe der vorliegenden Erfindung war die Bereitstellung von kristallinen Modifikationen von N-α-(2,4,6-Triisopropylphenylsulfonyl)-3-hydroxyamidino-(L)-phenylalanin-4-ethoxycarbonylpiperazid und/oder Sulfat-Salzen davon, welche gegenüber den Verbindungen aus dem Stand der Technik vorteilhafte Eigenschaften aufweisen.

Die vorliegende Erfindung stellt kristalline Modifikationen von N-α-(2,4,6-Triisopropylphenylsulfonyl)-3-hydroxyamidino-(L)-phenylalanin-4-ethoxy-carbonylpiperazid und/oder Sulfat-Salzen davon bereit.

Gemäß vorliegender Erfindung wurden neue kristalline Modifikationen des oben genannten Urokinase-Inhibitors gefunden, die gegenüber der amorphen Form dieser Verbindung entscheidende Vorteile aufweisen. Die erfindungsgemäßen kristallinen Modifikationen besitzen bedeutende Vorteile in der Handhabung, Lagerung und Formulierung.

Überraschenderweise kann die Verbindung N-α-(2,4,6-Triisopropylphenylsulfonyl)-3-hydroxyamidino-(L)-phenylalanin-4-ethoxy-carbonylpiperazid und/oder Sulfat-Salze davon kristallisiert werden, was bisher nicht möglich war, und ist daher in ihren Eigenschaften den amorphen Verbindungen aus dem Stand der Technik überlegen. So zeichnen sich die erfindungsgemäßen kristallinen Modifikationen durch eine sehr geringe Hygroskopie aus. Sie sind außerdem gegen Zersetzung sehr beständig und eignen sich daher auch für eine längere Lagerung. Außerdem weisen die erfindungsgemäßen kristallinen Modifikationen verbesserte Filtrations- und Trocknungseigenschaften auf. Darüber hinaus eignen sich die kristallinen Modifikationen von N-α-(2,4,6-Triisopropylphenylsulfonyl)-3-hydroxyamidino-(L)-phenylalanin-4-ethoxy-carbonylpiperazid und dessen Sulfat-Salzen ideal zur Formulierung in Arzneimittelzusammensetzungen.

Wie hierin verwendet, bedeutet WX-671 die freie Base von N-α-(2,4,6-Triisopropylphenylsulfonyl)-3-hydroxyamidino-(L)-phenylalanin-4-ethoxy-carbonylpiperazid. WX-671.1 bezeichnet das Hydrogensulfat der genannten Verbindung und WX-671.2 bezeichnet das Sulfat der genannten Verbindung.

Wie eingangs bereits ausgeführt, sind die erfindungsgemäße Modifikationen von WX-671 und Sulfat-Salzen davon im wesentlichen kristallin. Bisher war es nicht möglich, derartige Hydroxyamidin-Verbindungen in kristalliner Form herzustellen. Auch die im Stand der Technik bereits offenbarten Verbindungen waren nicht kristallisierbar.

Die erfindungsgemäßen kristallinen Modifikationen von N-α-(2,4,6-Triisopropylphenylsulfonyl)-3-hydroxyamidino-(L)-phenylalanin-4-ethoxy-carbonylpiperazid und dessen Salzen umfassen das Sulfatsalz (WX-671.2), sowie die freie Base (WX-671). Ferner wird das Hydrogensulfatsalz (WX-671.1) beschrieben.

Die erfindungsgemäßen kristallinen Modifikationen können Mischungen der freien Base und des Sulfat-Salzes umfassen. Vorzugsweise umfasst die erfindungsgemäße kristalline Modifikation jeweils entweder die freie Base oder das Sulfatsalz als kristalline Modifikation. Besonders bevorzugt umfasst die erfindungsgemäße kristalline Modifikation Einkristalle der jeweiligen kristallinen Modifikation.

Die erfindungsgemäßen kristallinen Modifikationen wurden durch Röntgendiffraktometrie untersucht und weisen vorzugsweise die in Tabelle 3.1 (für das Sulfatsalz WX-671.2), und Tabelle 11.1 (für die freie Base) dargestellten Peaks auf. Ferner weist die kristalline Modifikation des Hydrogen-Sulfat-Salzes die in Tabelle 7.1 (für das Hydrogensulfatsalz WX-671.1) dargestellten Peaks auf.

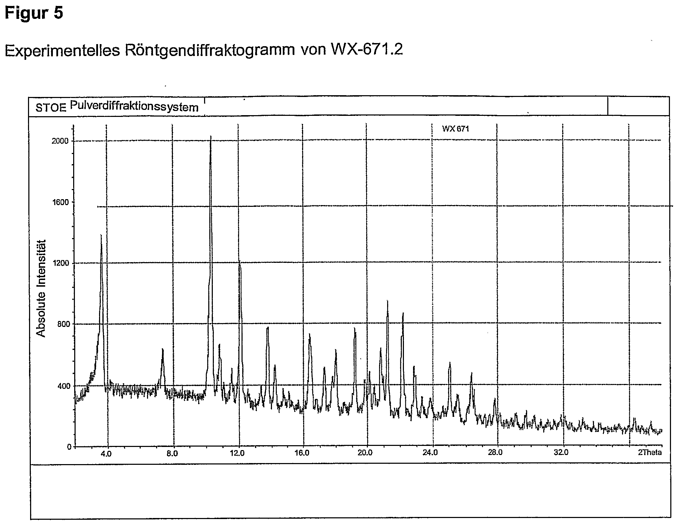

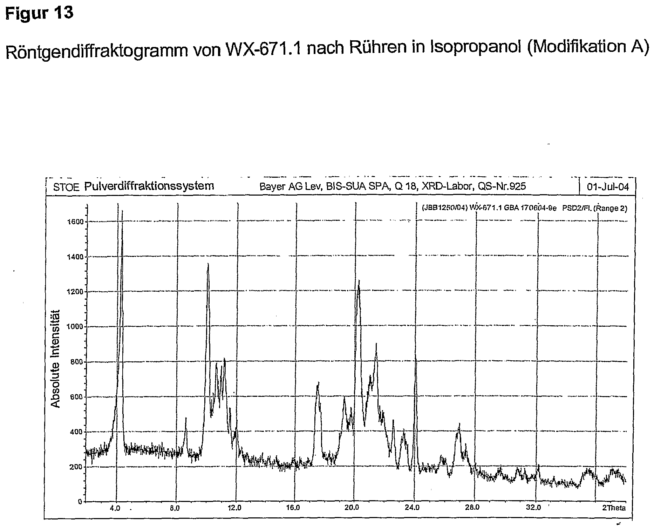

Die erfindungsgemäßen kristallinen Modifikationen weisen im Wesentlichen die in den Figuren 5 (für das Sulfatsalz WX-671.2), und 19 (für die freie Base) dargestellten Peaks auf. Die kristalline Modifikation des Hydrogen-Sulfat-Salzes weist im wesentlichen die in der Figur 13 (für das Hydrogensulfatsalz WX-671.1) dargestellten Peaks auf.

Wie in den Beispielen unten beschrieben ist, wurden zusätzlich zu den Röntgendiffraktometrie-Analysen auch thermoanalytische Untersuchungen durchgeführt (Differential Scanning Calorimetry, DSC und Thermogravimetrie, TGA). Dabei zeigte sich, dass die kristalline Verbindung des Hydrogensulfatsalzes WX-671.1 sich bei einer Temperatur von etwa 175 °C-195 °C zersetzt, genauer im Bereich ab etwa 185 °C (bei einer Heizrate von 2 Kmin⁻¹).

Die erfindungsgemäße kristalline Modifikation von WX-671.2 weist vorzugsweise N-α-(2,4,6-Triisopropylpheny(sulfonyl)-3-hydroxyamidino-(L)-phenylalanin-4-ethoxycarbonylpiperazid und Sulfat-Anionen in einem Molverhältnis von etwa 2:1 auf, wobei dieses Verhältnis in einem Bereich von 1,5 bis 2,5:1 variieren kann. Bevorzugt ist ein Verhältnis etwa 1,25 bis 2,25:1, stärker bevorzugt 1,1 bis 2,1:1 und am stärksten bevorzugt im Verhältnis von etwa 2:1.

Die kristalline Modifikation von WX-671.2 weist vorzugsweise Einheiten aus jeweils 2 Molekülen N-α-(2,4,6-Triisopropylphenylsulfonyl)-3-hydroxyamidino-(L)-phenylalanin-4-ethoxycarbonylpiperazid und einem Sulfat-Anion auf, wobei in jeder dieser Einheiten die 2 Moleküle N-α-(2,4,6-Triisopropylphenylsulfonyl)-3-hydroxyamidino-(L)-phenylalanin-4-ethoxycarbonylpiperazid in der gleichen Konformation vorliegen können oder sie können vorzugsweise auch in unterschiedlichen Konformationen vorliegen.

Zusätzlich liegt die kristalline Modifikation von WX-671.2 vorzugsweise als Hydrat, insbesondere als Trihydrat, vor, d.h. dass pro Mol Salz etwa 3 Mol Wasser vorhanden sind. Auch dieses Verhältnis kann natürlich geringen Schwankungen unterworfen sein, d.h. pro Mol Salz können im Durchschnitt zwischen 2,5 und 3,5 Mol Wasser vorhanden sein, vorzugsweise zwischen 2,25 und 3,25, stärker bevorzugt zwischen 2,2 und 3,2, stärker bevorzugt zwischen 2,1 und 3,1.

Das Sulfatsalz von N-α-(2,4,6-Triisopropylphenylsulfonyl)-3-hydroxyamidino-(L)-phenylalanin-4-ethoxycarbonylpiperazid hat sich, unabhängig von der Kristallinität, als thermodynamisch stabile Verbindung herausgestellt. Daher stellt die vorliegende Erfindung auch die neue Verbindung des N-α-(2,4,6-Triisopropylphenylsulfonyl)-3-hydroxyamidino-(L)-phenylalanin-4-ethoxycarbonylpiperazid-Sulfatsalzes bereit. Auch das nicht kristalline Sulfatsalz WX-671.2 eignet sich wie unten für die kristallinen Modifikationen beschrieben zur Herstellung von Arzneimitteln.

In der kristallinen Modifikation von WX-671.1 sind N-α-(2,4,6-Triisopropylphenylsulfonyl)-3-hydroxyamidino-(L)-phenylalanin-4-ethoxycarbonylpiperazid und Hydrogensulfat-Anionen in einem Molverhältnis von 0,5 bis 1,5:1, von 0,8 bis 1,2:1, von 0,9 bis 1,1:1, oder von etwa 1:1 vorhanden.

Eine kristalline Modifikation von WX-671.1 kann auch als Hydrat vorliegen.

Die kristalline Modifikation von WX-671 (freie Base) liegt vorzugsweise nicht als Hydrat vor. Eine Hydratform ist jedoch möglich.

Die erfindungsgemäßen kristallinen Modifikationen können gegebenenfalls mit geeigneten pharmazeutischen Hilfs- und/oder Trägerstoffen zur Herstellung von Arzneimitteln verwendet werden. Dabei ist eine Verabreichung in Kombination mit anderen Wirkstoffen, z.B. anderen Urokinase-Inhibitoren, wie etwa Antikörpern und/oder Peptiden, aber auch mit Chemotherapeutika und Zytostatika oder/und anderen zytostatischen und zytotoxischen Wirkstoffen möglich.

Die erfindungsgemäßen kristallinen Modifikationen können somit in einer geeigneten Arzneimittelformulierung zubereitet werden, beispielsweise als Tabletten, Dragees, Kapseln, Pastillen, Pulver, Sirup, Suspension, Lösung oder dgl. Bevorzugt ist insbesondere eine Arzneimittelzubereitung für die orale Verabreichung.

Die erfindungsgemäßen kristallinen Modifikationen sind zur Bekämpfung von Krankheiten geeignet, die mit einer pathologischen Überexpression von uPA oder/und Urokinase-Plasminogen-Aktivatorrezeptor (uPAR) assoziiert sind. Sie ist beispielsweise in der Lage, hocheffizient das Wachstum oder/und die Ausbreitung maligner Tumoren sowie die Metastasierung von Tumoren zu hemmen. Beispiele hierfür sind Tumorerkrankungen, z.B. Brustkrebs, Lungenkrebs, Blasenkrebs, Magenkrebs, Cervixkrebs, Ovarienkrebs, Nierenkrebs, Prostatakrebs und Weichgewebesarkome, insbesondere mit einer hohen Metastasierungsrate assoziierte Tumore.

Die erfindungsgemäßen Modifikationen können allein oder in Kombination mit anderen physiologisch wirksamen Substanzen eingesetzt werden, z.B. mit Radiotherapeutika oder mit zytotoxischen oder/und zytostatischen Mitteln, z.B. Chemotherapeutika, wie etwa cis-Platin, Doxorubicin, 5-Fluoruracil, Taxolderivaten, oder/und sonstigen chemotherapeutischen Agenzien, beispielsweise ausgewählt aus der Gruppe der alkylierenden Agenzien, Antimetaboliten, Antibiotika, Epidophyllotoxine und Vinca-Alkaloide. Ebenfalls möglich ist eine Kombination mit Strahlentherapie oder/und chirurgischen Eingriffen.

Weiterhin sind die erfindungsgemäßen kristallinen Modifikationen auch für andere uPA-assoziierte Erkrankungen wirksam. Beispiele für derartige Erkrankungen sind etwa pulmonärer Bluthochdruck und/oder Herzerkrankungen (z.B. WO 02/00248), Magen- und Darmerkrankungen, wie etwa inflammatorische Darmerkrankungen, prämaligne Colonadenome, entzündliche Erkrankungen, wie etwa septische Arthritis, oder andere Erkrankungen, wie Osteoporose, Cholesteatomie, Haut- und Augenerkrankungen, wie beispielsweise altersbedingte Makuladegeneration (AMD), sowie virale oder bakterielle Infektionen, wobei ausdrücklich auf die in EP-A-0 691 350, EP-A-1 182 207 und US-Patent 5,712,291 genannten Erkrankungen Bezug genommen wird.

Ein weiterer Gegenstand der vorliegenden Erfindung ist ein Arzneimittel, welches eine erfindungsgemäße kristalline Modifikation als Wirkstoff umfasst. Ein solches Arzneimittel kann gegebenenfalls zusätzlich pharmazeutisch verträgliche Träger und/oder Hilfsstoffe umfassen. Die Arzneimittel können beim Menschen oder Tieren topisch, oral, rektal oder parenteral, z.B. intravenös, subkutan, intramuskulär, intraperitoneal, oder auch sublingual, nasal und/oder inhalativ verabreicht werden. Geeignete Verabreichungsformen sind z.B. Tabletten, Dragees, Kapseln, Pastillen, Pellets, Pulver, Suppositorien, Lösungen, Sirup, Emulsionen, Suspensionen, Liposomen, Inhalationssprays oder transdermale Systeme, wie etwa Pflaster. Eine besonders bevorzugte Arzneimittelzusammensetzungen ist für eine orale Verabreichung geeignet, z.B. auch als Slow-Release-Retard.

Des Weiteren stellt die vorliegende Erfindung eine Verwendung der erfindungsgemäßen kristallinen Modifikationen zur Herstellung von pharmazeutischen Zusammensetzung für die Bekämpfung von Krankheiten bereit, welche mit einer pathologischen Überexpression von Urokinase und/oder dem Urokinase-Rezeptor assoziiert sind. Insbesondere eignet sich ein solches Arzneimittel mit dem erfindungsgemäßen Wirkstoff zur Tumorbehandlung und/oder -prävention und insbesondere auch zur Behandlung oder Prävention von Metastasenbildung, zur Behandlung von Primärtumoren und Sekundärtumoren.

Durch die vorliegende Erfindung wird eine Möglichkeit zur Urokinasehemmung bei Lebewesen, insbesondere dem Menschen, durch Verabreichung einer wirksamen Menge der erfindungsgemäßen Modifikation bereitgestellt. Die zu verabreichende Dosis hängt ab von der Art und Schwere der zu behandelnden Erkrankungen. Beispielsweise liegt die Tagesdosis im Bereich von 0,01-100 mg/kg Wirksubstanz pro Körpergewicht, stärker bevorzugt 0,1-50 mg/kg, stärker bevorzugt 0,5-40 mg/kg, stärker bevorzugt 1-30 mg/kg, stärker bevorzugt 5-25 mg/kg.

Ein weiterer Gegenstand der vorliegenden Erfindung ist ein Verfahren zur Herstellung einer im wesentlichen kristallinen Modifikation eines N-α-(2,4,6-Triisopropylphenylsulfonyl)-3-hydroxyamidino-(L)-phenylalanin-4-ethoxy-carbonylpiperazid-Salzes, umfassend die Schritte:
(a) Bereitstellen der Verbindung N-α-(2,4,6-Triisopropylphenylsulfonyl)-3-hydroxyamidino-(L)-phenylalanin-4-ethoxycarbonylpiperazid oder eines ihrer Salze,
(b) Lösen oder/und Suspendieren der Verbindung oder des Salzes derselben aus Schritt (a) in einem zur Bildung der kristallinen Modifikation geeigneten Lösungsmittel,
(c) Abtrennen der kristallinen Modifikation.

Es hat sich überraschenderweise gezeigt, dass die kristallinen Modifikationen von WX-671, WX-671.1 und WX-671.2 auf einfache Weise in kristalliner Form hergestellt werden können. Vorzugsweise wird als Ausgangsmaterial ein Salz von N-α-(2,4,6-Triisopropylphenylsulfonyl)-3-hydroxyamidino-(L)-phenylalanin-4-ethoxycarbonylpiperazid, z.B. das Hydrogensulfatsalz, verwendet.

Es kommen jedoch auch weitere Salzverbindungen als Ausgangsverbindungenzur Bildung kristalliner Modifikationen in Frage, beispielsweise das Besylatsalz, Hydrochloridsalz, Mesylatsalz, Tatratsalz und andere.

Als Lösungsmittel für Schritt (b) können vorzugsweise verschiedene organische Lösungsmittel verwendet werden. Geeignet sind beispielsweise Wasser sowie verschiedene Alkohole, z.B. Methanol, Ethanol, Propanol, Butanol und deren Isoformen, wie z.B. Isopropanol, Isobutanol etc., sowie weiterhin Glykole, Ether, Glykolether, Aceton und dgl. Weitere geeignete Lösungsmittel sind Tetrahydrofuran (THF) und Acetonitril. Bevorzugt sind als Lösungsmittel insbesondere Aceton und Acetonitril.

Es kann jedoch auch Wasser als Lösungsmittel verwendet werden. Insbesondere zur Umkristallisation (siehe Schritt (d)) wird vorzugsweise Wasser verwendet.

Wird zur Herstellung einer kristallinen Modifikation eines Salzes von N-α-(2,4,6-Triisopropylphenylsulfonyl)-3-hydroxyamidino-(L)-phenylalanin-4-ethoxycarbonylpiperazid als Ausgangsmaterial die freie Base verwendet, so werden zusätzlich in Schritt (b) geeignete Salze oder Säuren hinzugegeben, um das jeweilige gewünschte Salz von N-α-(2,4,6-Triisopropylphenylsulfonyl)-3-hydroxyamidino-(L)-phenylalanin-4-ethoxycarbonylpiperazid zu erhalten.

Das erfindungsgemäße Verfahren kann auch einen weiteren Schritt (d) des Umkristallisierens des kristallinen Verbindungen aus Schritt (c) umfassen.

Dies gilt insbesondere für die Herstellung von WX-671.2 aus WX-671.1. Hierbei wird vorzugsweise die in Schritt (c) entstandene kristalline Modifikation wiederum in einem geeigneten Lösungsmittel oder einer Mischung von Lösungsmitteln umkristallisiert. Auch hier können als Lösungsmittel die zuvor genannten eingesetzt werden. Insbesondere ist Wasser bevorzugt, bzw. es wird vorzugsweise mit einem zur Bildung der gewünschten kristallinen Modifikation ausreichenden Gehalts an Wasser umkristallisiert. Wasser ist insbesondere dann bevorzugt, wenn, wie z.B. im Fall von WX-671.2, ein Hydrat (im Fall von WX-671.2 ein Trihydrat) gebildet wird.

Die Erfindung soll durch die folgenden Figuren und die Beispiele näher erläutert werden.

### Beschreibung der Figuren

**Figur 1** zeigt einen Ortep-Plot (50 %) mit Bezeichnungschema für Molekül A (WX-671.2).
**Figur 2** zeigt einen Ortep-Plot (50 %) mit Bezeichnungschema für Molekül B (WX-671.2).
**Figur 3** zeigt unabhängige Moleküle innerhalb einer Elementarzelle einer Einheit aus zwei Molekülen WX-671.2 und Sulfatanion.

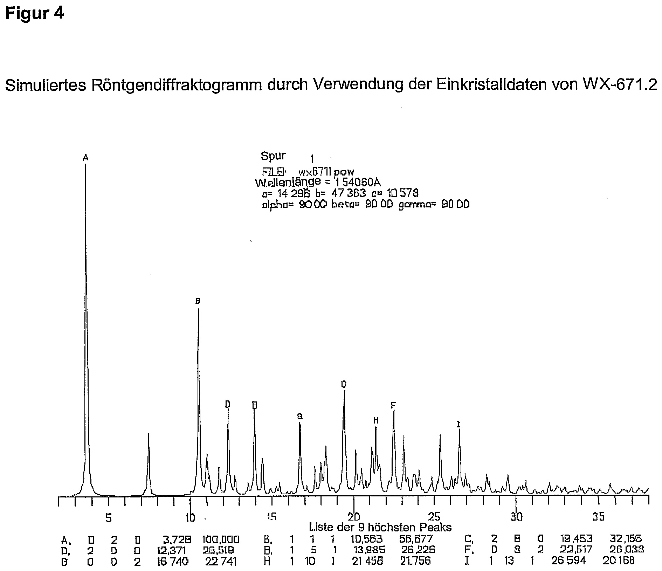

**Figur 4** zeigt ein simuliertes Röntgendiffraktogramm unter Verwendung von Einkristalldaten von WX-671.2.
**Figur 5** zeigt ein experimentelles Röntgendiffraktogramm von WX-671.2.

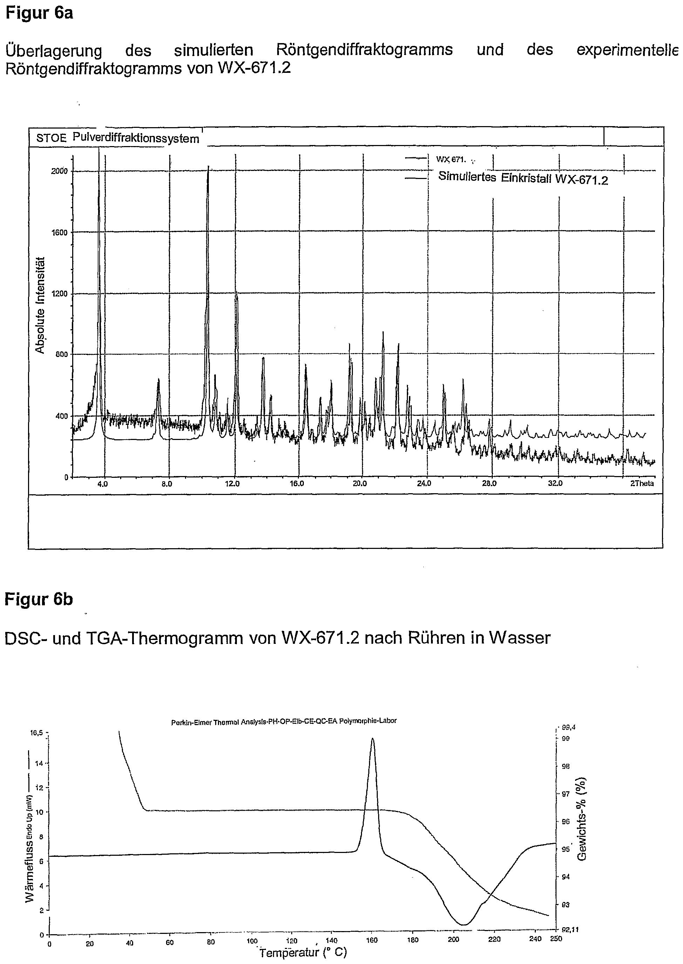

**Figur 6a** zeigt die Überlagerung des simulierten Röntgendiffraktionsmusters und des experimentellen Röntgendiffraktionsmusters gemäß der Figuren 4 und 5 für WX-671.2.
**Figur 6b** zeigt das DSC- und TGA-Thermogramm von WX-671.2 nach Rühren in Wasser.
**Figur 7** führt die Kristalldaten und Struktur-Refinement für WX-671.2 auf.
**Figur 8** zeigt die Bindungslängen [A] und Winkel [°] für WX-671.2.
**Figur 9** zeigt die Torsionswinkel [°] für WX-671.2.
**Figur 10** zeigt das DSC- und TGA-Thermogramm von WX-671.1 (Modifikation A).
**Figur 11** zeigt das DSC- und TGA-Thermogramm einer Probe (Mesophase B) von WX-671.1.
**Figur 12** zeigt das DSC- und TGA-Thermogramm der Mesophase C von WX-671.1.
**Figur 13** zeigt das Röntgendiffraktogramm der Modifikation A von WX-671.1.
**Figur 14** zeigt das Röntgendiffraktogramm der Mesophase B von WX-671.1.
**Figur 15** zeigt das Röntgendiffraktogramm der Mesophase C von WX-671.1.
**Figur 16** zeigt das DSC- und TGA-Thermogramm der amorphen Ausgangssubstanz der freien Base WX-671.
**Figur 17** zeigt das DSC- und TGA-Thermogramm einer aus Acetonitril im Gefrierschrank kristallisierten Probe der freien Base WX-671.
**Figur 18** zeigt eine mikroskopische Aufnahme der freien Base WX-671 nach Kristallisation aus Acetonitril im Gefrierschrank.
**Figur 19** zeigt das Röntgendiffraktogramm der freien Base WX-671 nach Kristallisation aus Acetonitril im Gefrierschrank.
**Figur 20** zeigt die Sorptionsisotherme von Wasserdampf an die freie Base WX-671 bei 22 °C.

### Beispiele

### Beispiel 1

### Herstellung verschiedener Salze von N-α-(2,4,6-Triisopropyl-phenylsulfonyl)-3-hydroxyamidino-(L)-phenylalanin-4-ethoxy-carbonyl-piperazid

Die Salze wurden durch Lösen von 6.0 g WX-671 in 50 ml Aceton hergestellt.

Die verwendeten Säuren wurden ohne Verdünnung in 25prozentigem Überschuss hinzugegeben und für zwei Stunden bei Raumtemperatur gerührt.

### Kristallisationsbedingungen

**Tabelle 1.1**

| Säure | Äquivalente | Kristallisationsbedingungen | Trocknung |
|---|---|---|---|
| HCl | 1.25 mol eq. | Klare Lösung | Hochvakuum |
| H₂SO₄ | 1.25 mol eq. | Aus Acetonlösung kristallisiert | Hochvakuum |
| MsOH | 1.25 mol eq. | Aus Acetonlösung kristallisiert | Hochvakuum |
| BsOH | 1.25 mol eq. | Klare Lösung | Hochvakuum |
| Weinsäure | 1.25 mol eq. | Klare Lösung | Hochvakuum |

| | | | |
|---|---|---|---|
| MsOH: Methansulfonsäure; BsOH: Benzolsulfonsäure | | | |

In einem zweiten Schritt wurden die Salze für 7 Tage in einem geeigneten Lösungsmittel suspendiert, filtriert und bei Raumtemperatur getrocknet.

### Untersuchung der Kristallinität

Verfahren: Röntgendiffraktometrie (XRD); Mikroskopie

**Tabelle 1.2**

| Spezies | XRD | Mikroskopie |
|---|---|---|
| Freie Base | überwiegend kristallin | Kleine Partikel |
| Besylat | überwiegend amorph | Agglomerate |
| Hydrochlorid | komplett amorph | Kleine Partikel |
| Mesylat | flüssigkristallin | Agglomerate |
| Hydrogensulfat | flüssigkristallin | Agglomerate |
| Tartrat | komplett amorph | Glas |

### Untersuchung der Hygroskopizität

Verfahren: Lagerung 1 Woche/85 % relative Feuchtigkeit; thermogravimetrische Analyse (TGA)

**Tabelle 1.3**

| Spezies | TGA |
|---|---|
| Freie Base | 1,5 % (semihydrat) |
| Mesylat | 10,1 % (adsorptiv) |
| Hydrochlorid | 7,5 % (adsorptiv) |
| Mesylat | 7,8 % (adsorptiv) |
| Hydrogensulfat | 0,8 % (adsorptiv) |
| Tartrat | 10,5 % (adsorptiv) |

### Beispiel 2

### Kristallisation und Einkristall Röntgenstrukturanalyse von WX-671.2

### (Sulfatsalz)

Die neue Form von N-α-(2,4,6-Triisopropylphenylsulfonyl)-3-hydroxyamidino-(L)-phenylalanin-4-ethoxy-carbonyl-piperazid-Sulfat wird erhalten durch Suspendieren des Hydrogensulfatsalzes (WX-671.1) der Verbindung WX-671 in Wasser und durch Abtrennen der sich bildenden kristallinen Verbindung.

Kristallines WX-671.2 wurde auf zwei verschiedene Arten erhalten:
a) Ca. 50 mg WX-671.1 wurden in ca. 0,5 ml Wasser suspendiert. Die Suspension wurde bei Raumtemperatur stehengelassen. Nach 6 Tagen wurde die Suspension filtriert und der Rückstand bei Raumtemperatur an der Luft getrocknet.
b) Ca. 0,2 g WX-671.1 wurden in ca. 2 ml Wasser suspendiert. Die Suspension wurde bei 25 °C geschüttelt. Nach 3 Tagen wurden der Rückstand abfiltriert und die Kristalle von WX-671.2 bei Raumtemperatur an der Luft getrocknet.

Aus dem hierbei hergestellten kristallinen Material (WX-671.2) wurde die Röntgenstruktur bestimmt. Es wurden hierbei auch Einkristalle erhalten.

Die Kristallstrukturbestimmung wurde durchgeführt unter Verwendung eines Bruker-Nonius Diffraktometers ausgestattet mit einem Proteum-CCD Flächendetektor, einer FR591 rotierenden Anode mit CuKα-Strahlung, Montel Spiegel als Monochromator und einem Kryoflex Niedrigtemperaturgerät (T=90K). Vollsphären Datenermittlung omega und phi Scans. Verwendete Programme: Datensammlung Proteum V.1.37 (Bruker-Nonius 2002), Datenreduzierung Saint+Version 6.22 (Bruker-Nonius 2001) und Absorptionskorrektur SADABS V.2.03 (2002). Kristallstrukturauflösung wurde erreicht mittels direkte Methoden wie in SHELXTL Version 6.10 (Sheldrick, Universität Göttingen) implementiert und mittels XP-Programm visualisiert.

Durch differenzielle Fourier Synthese wurden fehlende Atome lokalisiert und der Atomliste hinzugefügt. "Least squares refinement" auf F2 über alle gemessenen Intensitäten wurden mit dem Programm SHELXTL Version 6.10 (Sheldrick, Universität Göttingen, 2000) vorgenommen. Alle Nicht-Wasserstoffatome wurden "refined" unter Einschluss der "anisotropic displacement parameters".

**Tabelle 2.1**

| Chiralitäts Check* | Richtige Struktur | Invertierte Struktur |
|---|---|---|
| Flack Parameter (Standardabweichung) | 0.0298 (0.0282) | 0.9694 (0.0307) |
| Twin Basf (Standardabweichung) | 0.03 (3) | 0.97 (3) |
| wR2-Wert (mit Flack Parameter) | 0.2016 | 0.2219 |
| Chiralität | ***S***(C9) | ***R***(C9) |

| | | |
|---|---|---|
| * H. D. Flack, Acta Cryst., 1983, A39, 876-881 H. D. Flack, G. Bernardinelli, Acta Cryst., 1999, A55, 908-915 H. D. Flack, G. Bernardinelli, J. Appl. Cryst., 2000, 33, 1143-1148 | | |

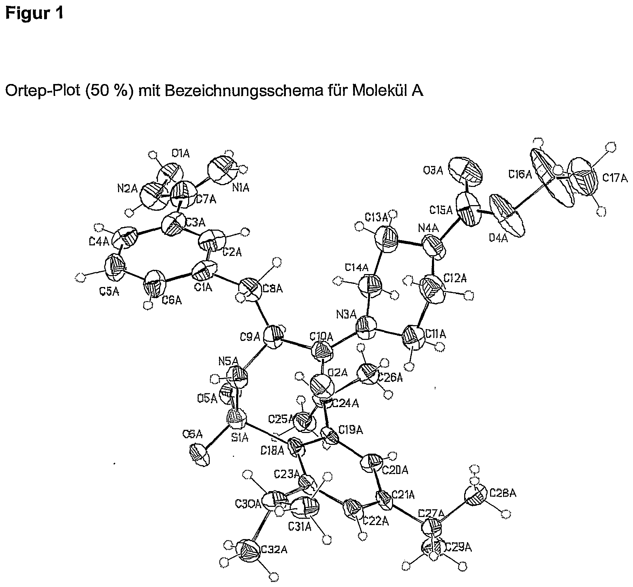

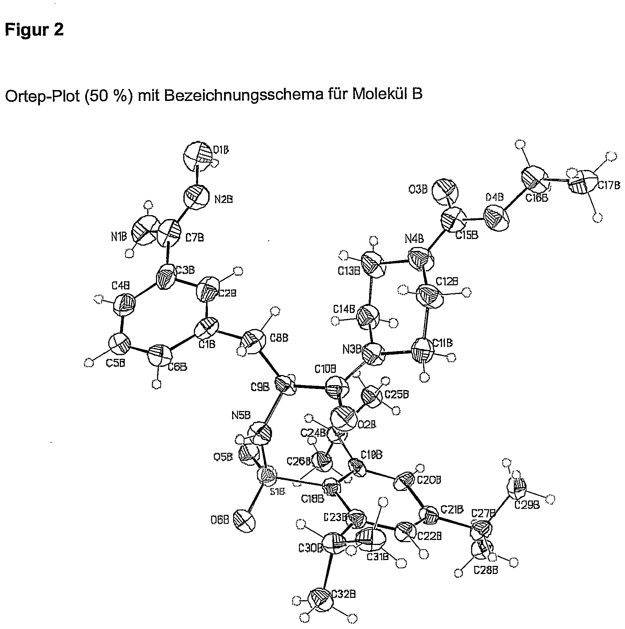

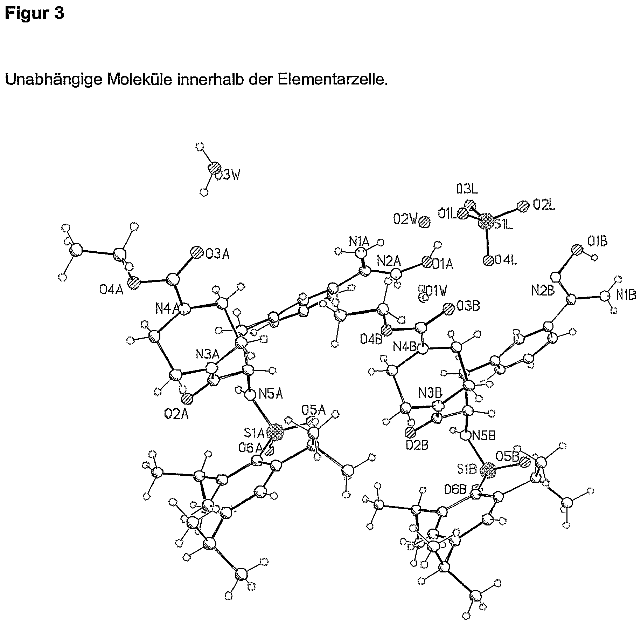

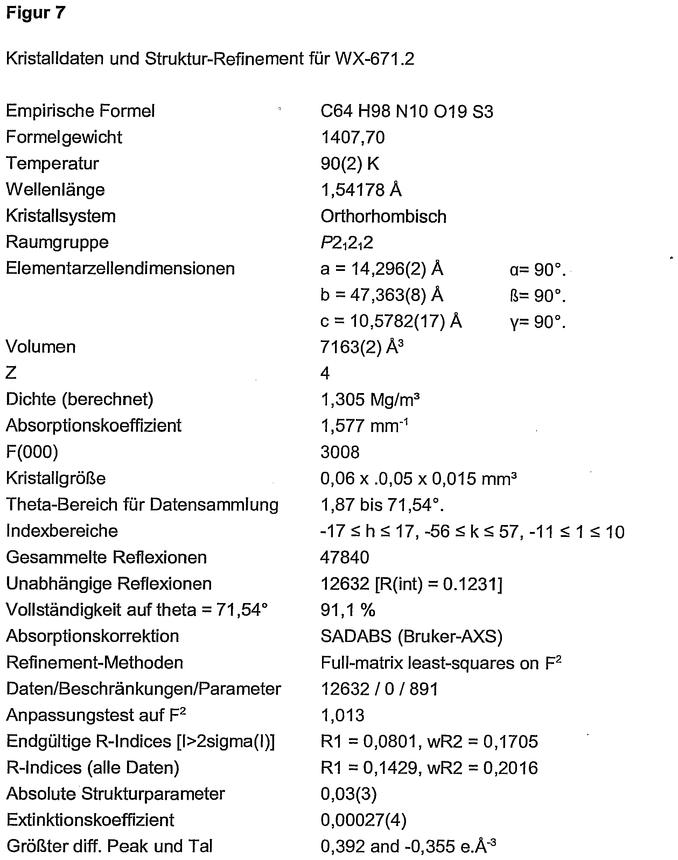

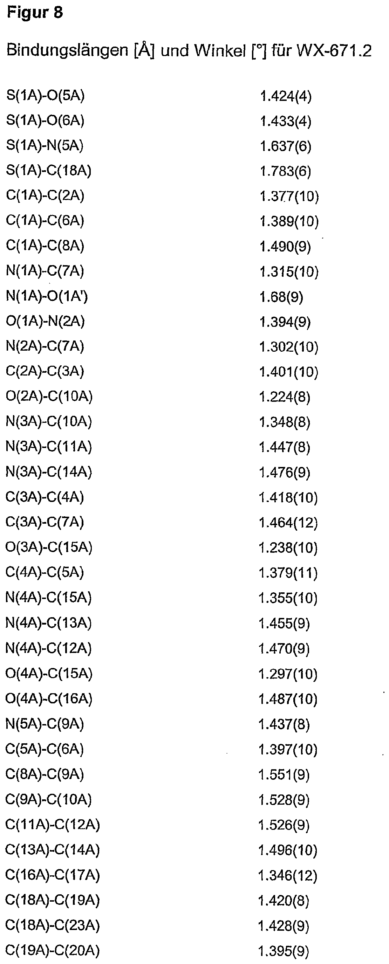

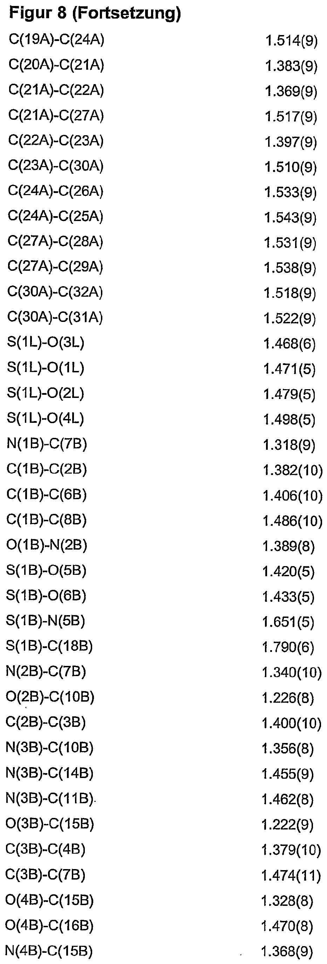

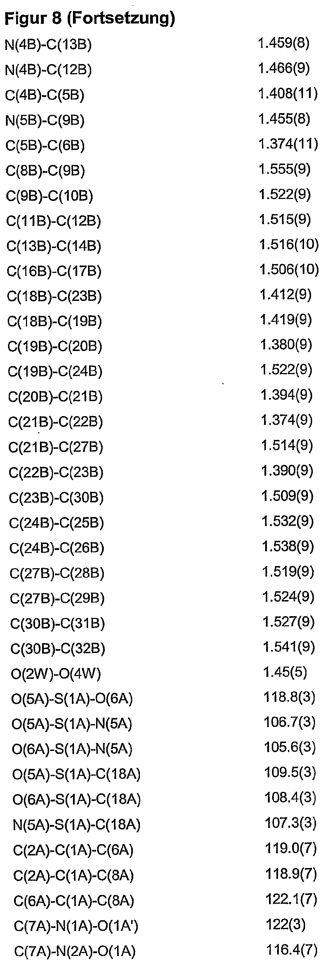

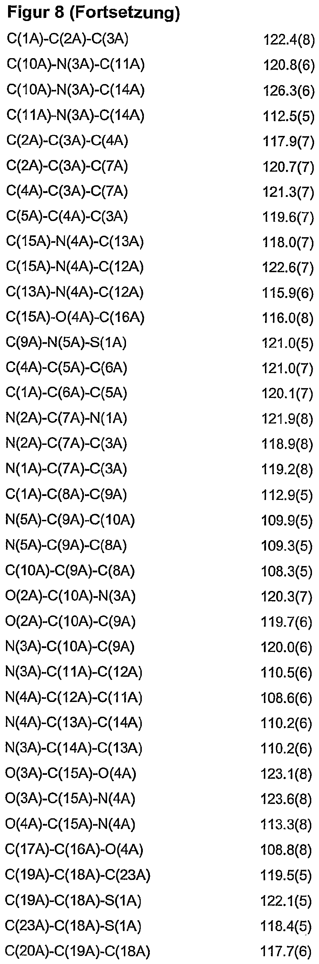

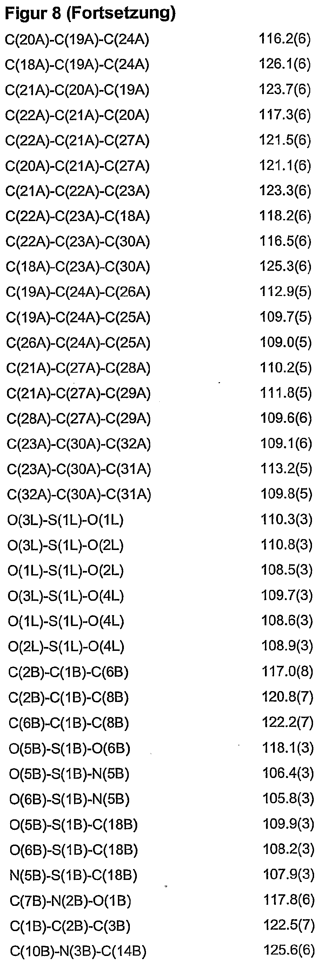

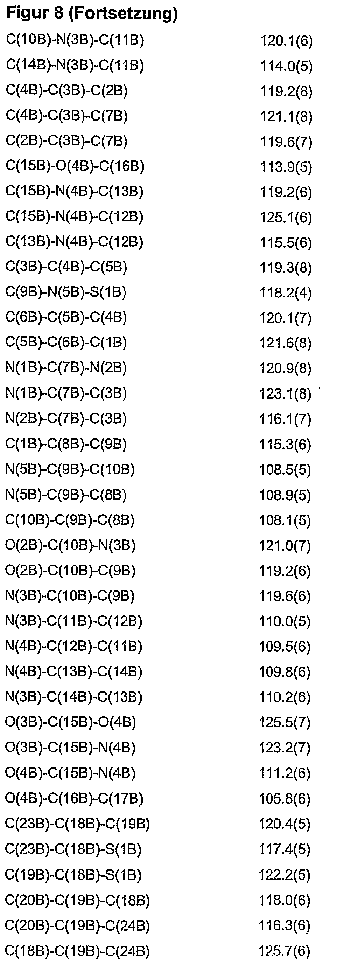

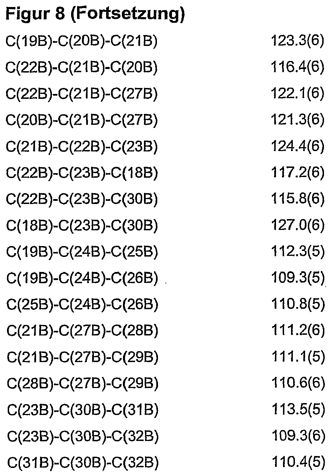

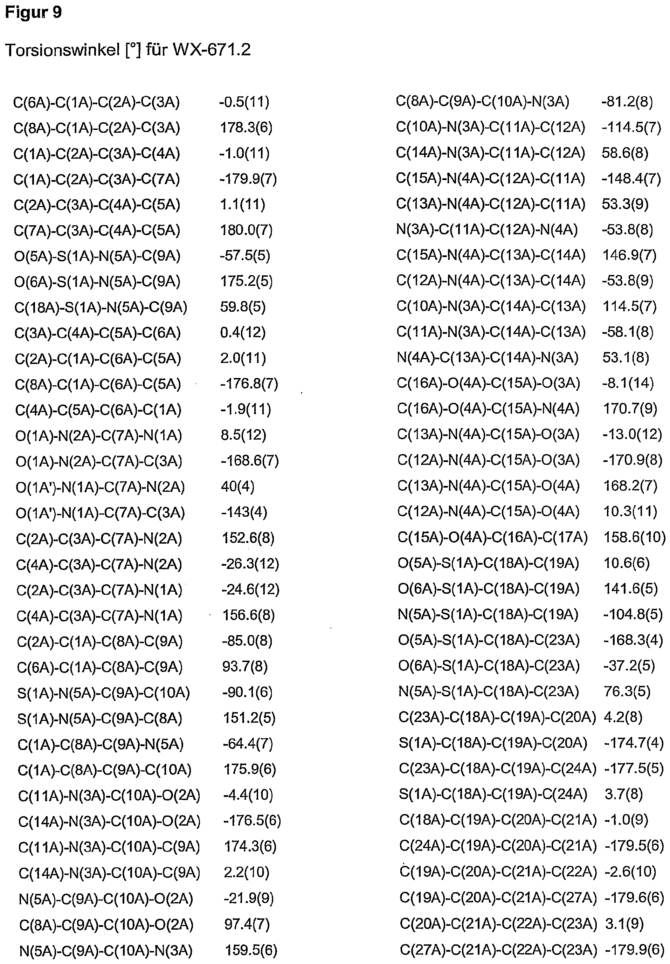

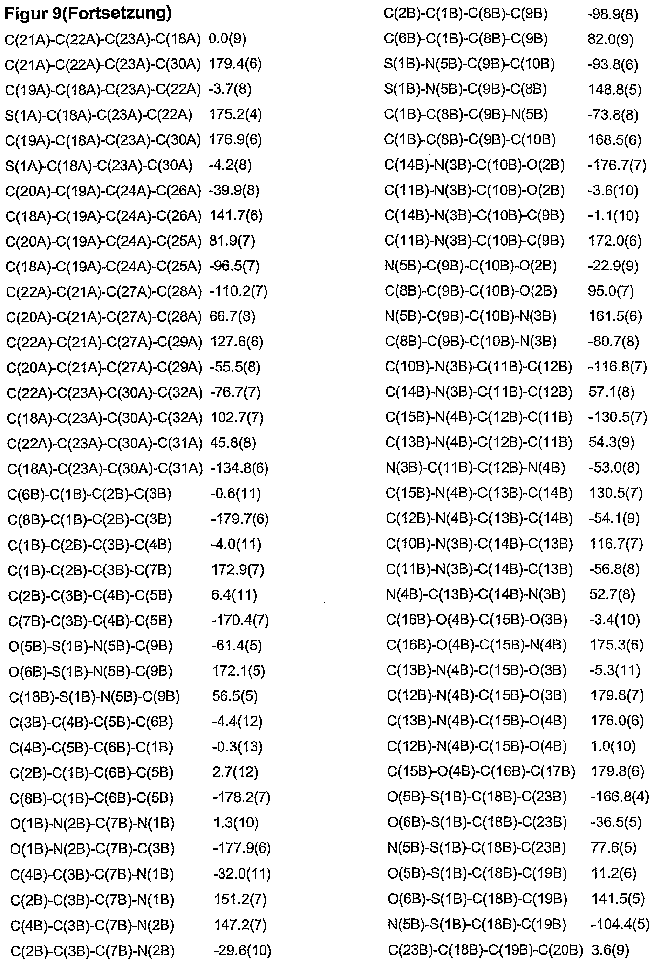

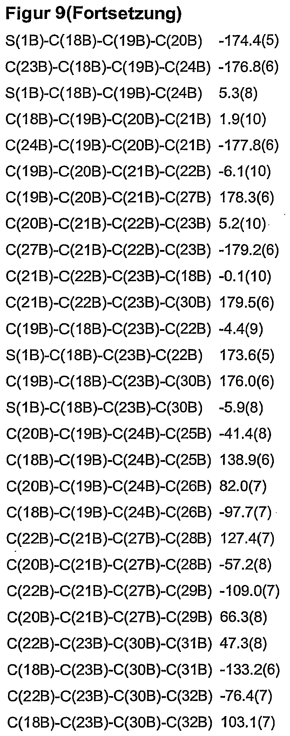

Die Ergebnisse der Röntgenstrukturanalyse sind in Figuren 1 bis 3 und 7 bis 9 dargestellt.

### Beispiel 3

### Röntgendiffraktometrie von WX-671.2 (Sulfatsalz)

Röntgendiffraktogramme wurden unter Verwendung eines Debye-Scherrer Diffraktometers STOE STADI-P erhalten, ausgestattet mit einem positionssensitiven Detektor (PSD, 5 °), einem Germanium [1 1 1 ]-primär Monochromator und einer CuKα 1,6 kW Keramik Röntgenröhre (1,5406 Å). Verwendetes Programm: Stoe WinXpow, Version 2.03 (2003).

**Tabelle 3.1**

| **Röntgendiffraktometrie-Peakliste für WX-671.2** | |
|---|---|
| **WX 671.2** | |
| Reflexe 2 Theta | Reflexe 2 Theta |
| 3,7 | 26,8 |
| 4,2 | 27,2 |
| 7,1 | 27,5 |
| 7,4 | 27,8 |
| 10,3 | 28,1 |
| 10,8 | 29,1 |
| 11,1 | 29,7 |
| 11,6 | 30,2 |
| 12,1 | 30,6 |
| 12,6 | 31,1 |
| 13,3 | 31,4 |
| 13,8 | 31,9 |
| 14,2 | 32,1 |
| 14,8 | 33,2 |
| 15,1 | 33,8 |
| 15,7 | 34,2 |
| 16,4 | 36,3 |
| 16,8 | 37,3 |
| 17,3 | |
| 17,8 | |
| 18,0 | |
| 18,6 | |
| 19,2 | |
| 19,8 | |
| 20,1 | |
| 20,4 | |
| 20,8 | |
| 21,0 | |
| 21,2 | |
| 21,8 | |
| 22,2 | |
| 22,9 | |
| 23,4 | |
| 23,9 | |
| 24,6 | |
| 25,0 | |
| 25,5 | |
| 26,2 | |
| 26,3 | |
| 26,5 | |

### Vergleichs-Beispiel 4

### Umkristallisationen von WX-671.1 (Hydrogensulfatsalz)

WX-671.1 wurde in Solventien (Isopropanol, Ethanol, Methanol) unterschiedlicher Polarität gelöst. Die Lösungen wurden filtriert, geviertelt und die Lösungsmittel mit unterschiedlicher Geschwindigkeit entfernt.

WX-671.1 existiert in seiner kristallinen Form (Modifikation A) und zwei Mesophasen B und C. Er zersetzt sich ab ca. 185 °C (Modifikation A und Mesophase B) bzw. 156 °C (Mesophase C). Zum Nachweis der Kristallinität von Modifikation A wird eine Röntgenstrukturbestimmung durchgeführt. Modifikation A ist die thermodynamisch stabile Form bei Raumtemperatur.

### Vergleichs-Beispiel 5

### Differential Scanning Calorimetry (DSC) und Thermogravimetrie (TGA) von WX-671.1 (Hydrogensulfatsalz)

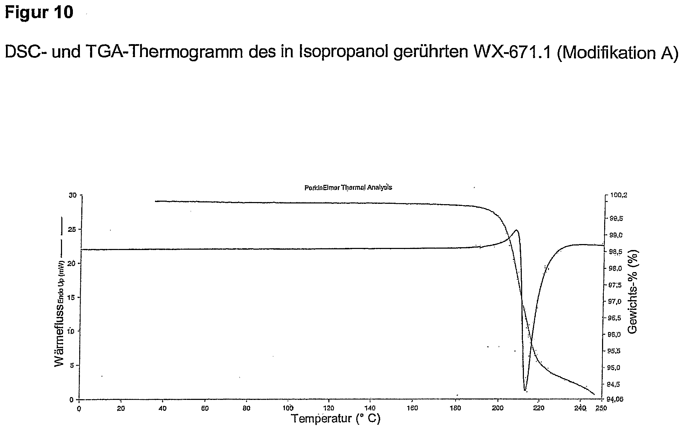

In diesem Beispiel wurden Thermogramme durch Differential Scanning Calorimetry (DSC) und Thermogravimetrie (TGA) erstellt. Die Figur 10 zeigt das DSC- und TGA-Thermogramm des eine Woche bei 25 °C in Isopropanol gerührten WX-671.1 (Modifikation A). Er zersetzt sich ab ca. 180 °C (exothermer Peak im DSC-Thermogramm, Masseverlust im TGA-Thermogramm im entsprechenden Temperaturbereich). Die Zersetzungstemperaturen sind stark Heizraten-abhängig und wurden im DSC-Kalorimeter bei einer Heizrate von 2 Kmin⁻¹ bestimmt. Die abgebildeten DSC-Übersichtsmessungen wurden mit einer Heizrate von 2 Kmin⁻¹ aufgenommen. Die Zersetzung wird entsprechend bei höheren Temperaturen registriert.

Bei 150 °C wird kein signifikanter Masseverlust registriert. Der schwach ausgeprägte endotherme Effekt vor dem Zersetzungspeak könnte durch teilweises Schmelzen oder partielle Umwandlung hervorgerufen werden.

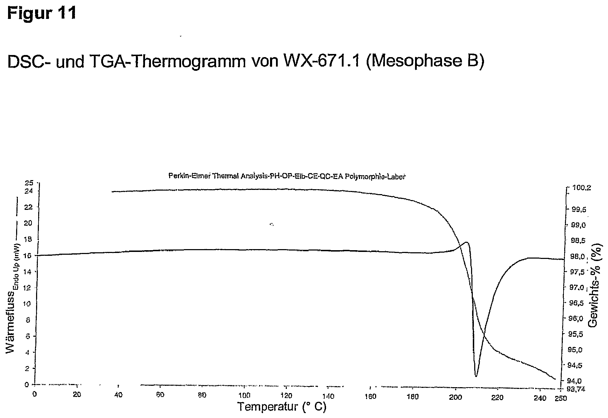

Figur 11 zeigt das DSC-Thermogramm einer für dieses Screening eingesetzten Probe von WX-671.1 (Mesophase B). Die Modifikation A und die Mesophase B sind thermoanalytisch identisch. Die Mesophase A wurde aus Isopropanol (Raumtemperatur/Kühlschrank) und Ethanol (Raumtemperatur) erhalten.

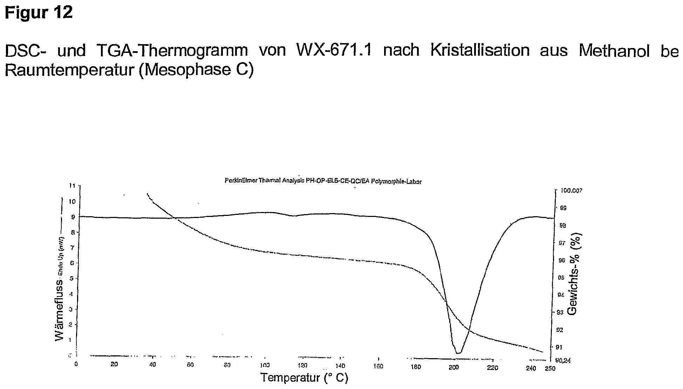

Die Figur 12 zeigt das DSC- und TGA-Thermogramm des Wirkstoffs nach einem Kristallisationsversuch aus Methanol bei Raumtemperatur (Mesophase C). Er zersetzt sich ab ca. 156 °C (Heizrate 2 Kmin⁻¹). Bis 175 °C wird eine Masseverlust von 4,5 % registriert. Im DSC-Thermogramm fehlt der endotherme Effekt vor dem Zersetzungspeak. Diese Form liegt in einem weniger geordneten Zustand vor als die Modifikation A und die Mesophase B. Sie ist nach Kristallisationsversuchen aus Methanol (Raumtemperatur, Kühlschrank) und Ethanol (Kühlschrank) entstanden. Bei den Proben wurden Masseverluste zwischen 4,5 und 4,8 Gew.-% registriert. Der stöchiometrische Wert für den Masseverlust von 2 Molekülen Wasser pro Wirkstoffmolekül beträgt 4,7 %. Bei dieser Form handelt es sich jedoch nicht um ein Hydrat.

### Vergleichs-Beispiel 6

### Thermomikroskopie von WX-671.1 (Hydrogensulfatsalz)

Von einer Probe von WX-671.1 wurden thermomikroskopische Aufnahmen getätigt (nicht dargestellt). Es wurden Agglomerate beobachtet, die keinen spezifischen Habitus zeigen. Der Wirkstoff zersetzt sich unter Blasenbildung ab ca. 197 °C. Unterschiede zur im DSC-Kalorimeter beobachteten Zersetzungstemperatur kommen durch die unterschiedlichen Heizraten zustande. Aus Lösungsmitteln wird WX-671.1 in unspezifischer Form erhalten. Der Wirkstoff zeigt z.T. Doppelbrechung, wie es für kristalline und mesomorphe Substanzen charakteristisch ist.

### Vergleichs-Beispiel 7

### Röntgendiffraktometrie von WX-671.1 (Hydrogensulfatsalz)

Das Röntgendiffraktogramm der Modifikation A (Figur 13) zeigt das für kristalline Phasen charakteristische Muster von zahlreichen scharfen Peaks bei höheren 2-Theta-Winkeln. Zur Bestätigung der Existenz einer kristallinen Phase werden weitere Untersuchungen, z.B. eine Röntgenstrukturanalyse, durchgeführt.

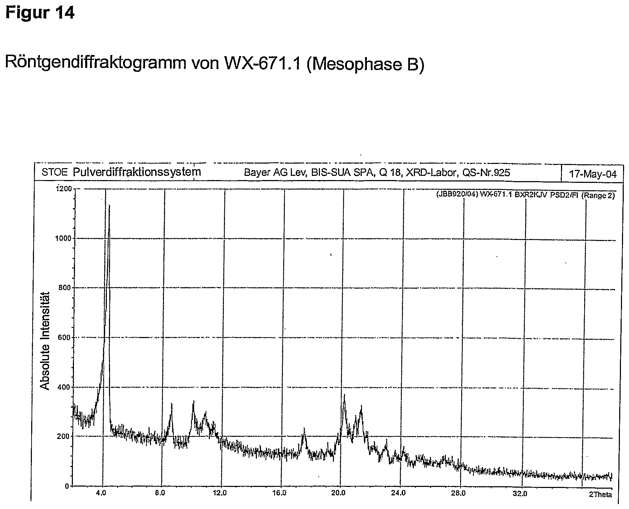

Im Röntgendiffraktogramm der Mesophase B (Figur 14) wurde ein scharfer Peak bei einem 2-Theta-Winkel von ca. 5° und weitere Reflexe mit geringer Intensität zwischen ca. 8° und ca. 25° beobachtet. Lage und Anzahl der Peaks ähneln denen der Modifikation A. Der bei niedrigem Theta-Winkel vorhandene Peak deutet auf das Vorhandensein einer Fernordnung der Moleküle hin, die Peaks mit geringer Intensität belegen das Vorhandensein einer Nahordnung. Daraus lässt sich schließen, dass die Mesophase B weder in kristalliner noch in amorpher Form, sonder vermutlich als Mesophase vorliegt.

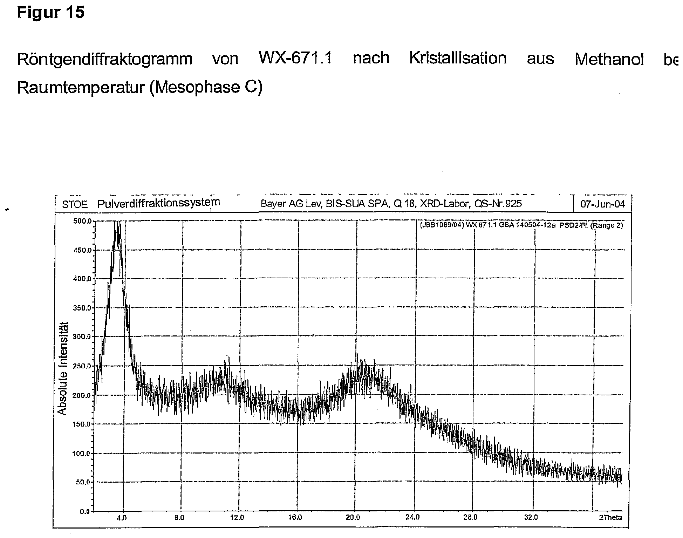

Das Röntgendiffraktogramm der Mesophase C (Figur 15) zeigt ebenfalls ein für mesomorphe Verbindungen charakteristisches Muster: einen intensiven Peak bei niedrigem 2-Theta-Winkel. Das Röntgendiffraktogramm belegt, dass Mesophase C keine kristalline Phase ist und wegen der vorhandenen Fernordnung nicht der amorphen Phase zugerechnet werden kann. Ein Vergleich mit dem Röntgendiffraktogramm von Mesophase B zeigt, dass Mesophase C vermutlich eine Phase mit einem geringeren Ordnungsgrad bildet. Ein Hinweis darauf, sind die bei Mesophase B stärker ausgeprägten und intensiveren Reflexe zwischen den 2-Theta-Winkeln von ca. 8° und ca. 25°.

Durch einwöchiges Rühren in Isopropanol bei Raumtemperatur wandelt sich die Mesophase B in die Modifikation A um. Durch Rühren in Wasser:Ethanol (1:1) verändert sie sich nicht. Durch mechanischen Stress (Mörsern, Pressen mit 9 kbar) wandelt sie sich ebenfalls nicht um.

**Tabelle 7.1**

| **Röntgendiffraktometrie-Peakliste für kristalline Modifikation von WX-671.1 (Hydrogensulfatsalz)** | |
|---|---|
| **WX 671.1** | |
| Reflexe 2 Theta | |
| 4,3 | |
| 8,6 | |
| 10,1 | |
| 10,3 | |
| 10,6 | |
| 11,0 | |
| 11,2 | |
| 11,3 | |
| 11,6 | |
| 12,0 | |
| 12,3 | |
| 13,8 | |
| 14,8 | |
| 15,9 | |
| 17,5 | |
| 17,7 | |
| 19,3 | |
| 19,7 | |
| 20,2 | |
| 21,0 | |
| 21,4 | |
| 21,7 | |
| 21,9 | |
| 22,6 | |
| 23,3 | |
| 24,0 | |
| 25,8 | |
| 26,9 | |
| 27,4 | |
| 28,0 | |
| 29,7 | |
| 30,8 | |
| 31,3 | |
| 32,2 | |
| 33,3 | |
| 35,5 | |
| 37,1 | |

### Beispiel 8

### Umkristallisationen von WX-671 (freie Base)

Der Wirkstoff WX-671 wird thermoanalytisch (DSC, TGA), durch Röntgendiffraktometrie sowie durch Kristallisationen aus organischen Solventien auf Polymorphie untersucht. WX-671 kristallisiert in einer Modifikation (Modifikation A). Der Wirkstoff zeigt eine sehr geringe Kristallisationstendenz.

Die Modifikation A die thermodynamisch stabile Form bei Raumtemperatur. Eine abschließende Bewertung der Polymorphie und Pseudopolymorphie von WX-671 ist erst nach Durchführung einer Polymorphiestudie möglich.

WX-671 wurde in Solventien (Tetrahydrofuran, Acetonitril, Methanol) unterschiedlicher Polarität gelöst. Die Lösungen wurden filtriert, geviertelt und der Wirkstoff mit unterschiedlicher Geschwindigkeit kristallisiert. Nach Trocknung bei Raumtemperatur wurden die Thermogramme (DSC, TGA) und die Röntgendiffraktogramme registriert.

Die für das Polymorphiescreening eingesetzte Ausgangssubstanz konnte in überwiegend kristalliner Form hergestellt werden. Die Kristallisation gelang aus Acetonitril im Gefrierschrank bei ca -18 °C.

### Beispiel 9

### Differential Scanning Calorimetry (DSC) und Thermogravimetrie (TGA) von WX-671 (freie Base)

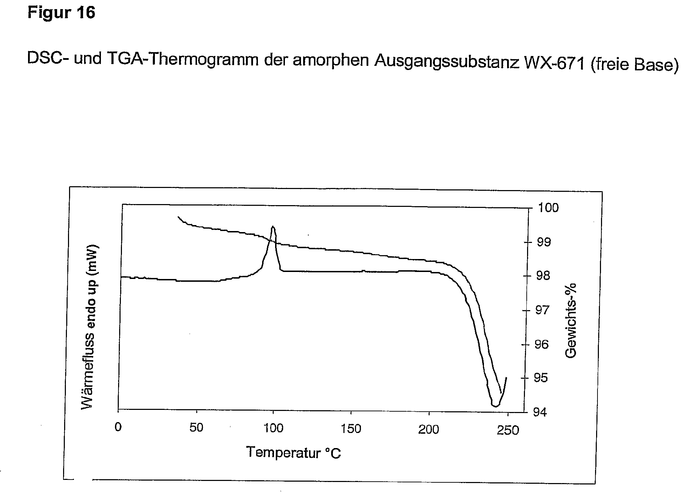

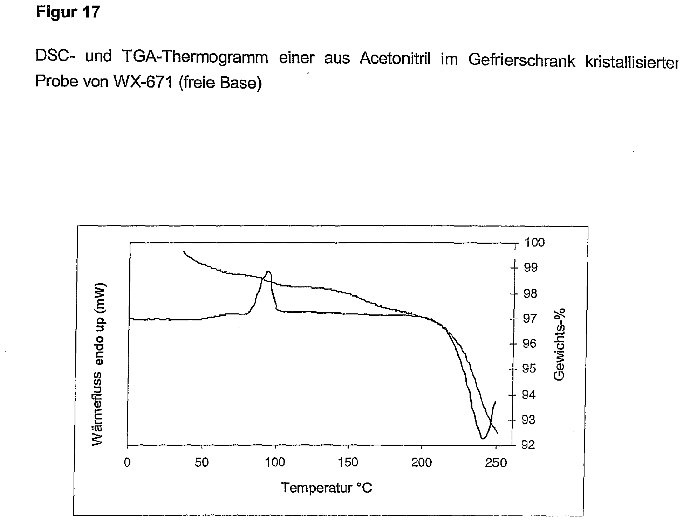

Die Figur 16 zeigt das DSC- und TGA-Thermogramm der amorphen Ausgangssubstanz. Ebenso wie die DSC- /TGA-Thermogramme des größten Teils der übrigen untersuchten Proben zeigt es einen thermischen Effekt im Bereich zwischen 45 °C und 85 °C. Im Rahmen des Polymorphiescreenings konnte dieser thermische Effekt nicht zugeordnet werden. Es könnte sich dabei um eine Glasumwandlung handeln. Daraus ergeben sich Rückschlüsse auf eine Kristallform mit einem Schmelzpunkt von ca. 190 °C. Die Substanz zersetzt sich in Abhängigkeit von der Heizrate ab ca. 155 °C. Aus diesem Grund kann der Schmelzpunkt der kristallinen Probe (GBA 190903-8c) nicht ermittelt werden. Die Thermogramme der amorphen und kristallinen Substanz sind deshalb weitgehend identisch (Figuren 16 und 17).

### Beispiel 10

### Mikroskopie von WX-671 (freie Base)

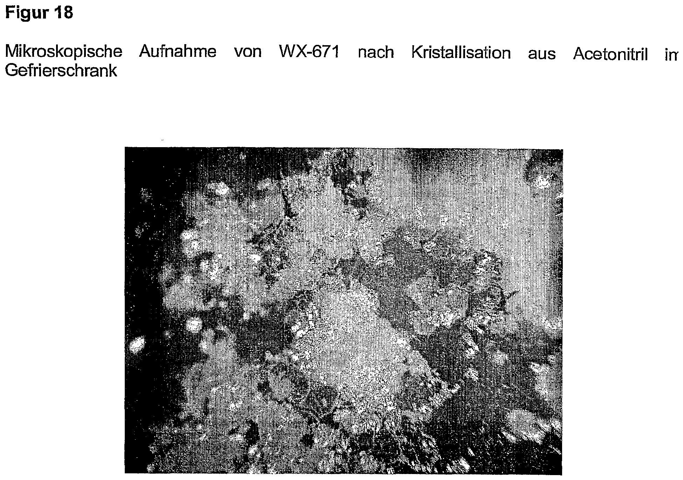

Aus Acetonitril kristallisiert WX-671 im Gefrierschrank in Form von Prismen (Figur 18). Aus anderen Lösungsmitteln kristallisiert der Wirkstoff nicht oder nur zu sehr geringem Anteil, aus der Schmelze kristallisiert er nicht.

### Beispiel 11

### Röntgendiffraktometrie von WX-671 (freie Base)

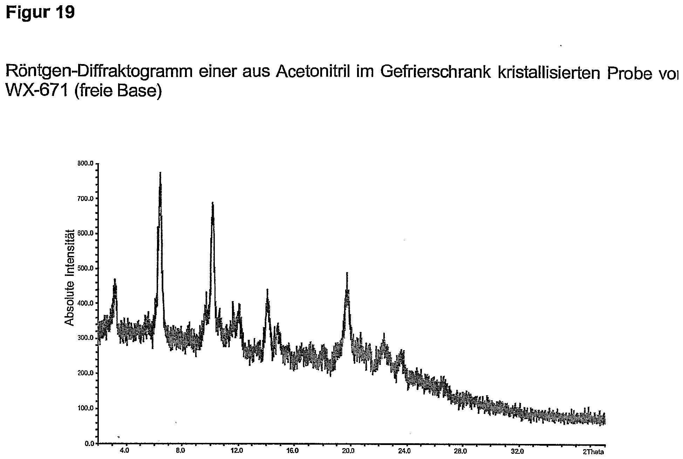

Die Figur 19 zeigt das Röntgendiffraktogramm des Wirkstoffs nach Kristallisation aus Acetonitril im Gefrierschrank (Modifikation A).

**Tabelle 11.1**

| **Röntgendiffraktometrie-Peakliste für WX-671 (freie Base)** |
|---|
| Probe wurde aus Acetonitril im Gefrierschrank kristallisiert. |
| **WX 671** |
| Reflexe 2 Theta |
| 3,2 |
| 5,5 |
| 6,4 |
| 8,5 |
| 9,7 |
| 10,2 |
| 10,7 |
| 11,2 |
| 11,5 |
| 11,7 |
| 12,1 |
| 13,4 |
| 13,8 |
| 14,1 |
| 14,6 |
| 14,8 |
| 15,5 |
| 16,5 |
| 18,1 |
| 19,1 |
| 19,7 |
| 20,5 |
| 20,7 |
| 21,3 |
| 22,4 |
| 22,7 |
| 23,6 |

### Beispiel 12

### Feuchtesorption von WX-671 (freie Base)

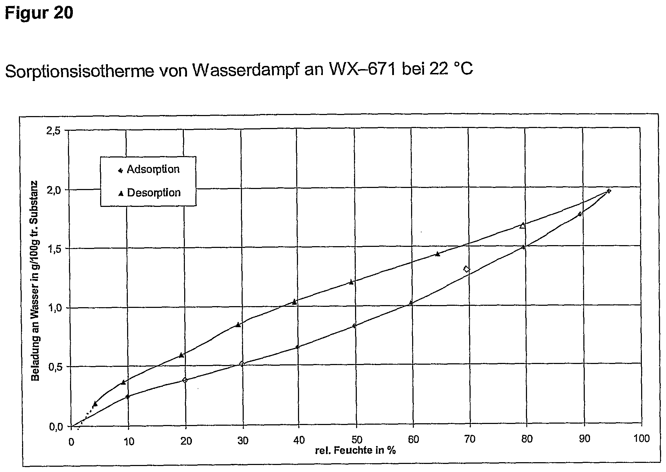

Figur 20 zeigt die Sorptionsisotherme von Wasserdampf an WX-671 bei 22 °C. Der amorphe Wirkstoff nimmt von 0 % r.F. Bis 95 % r.F. kontinuierlich Wasser auf. Bei der Trocknung wird dieses Wasser wieder abgegeben. Bei den meisten Punkten der Isotherme wurde innerhalb der jeweiligen Haltezeit kein Gleichgewichtszustand erreicht. Das Probengewicht stieg bzw. fiel weiter. Es gab in diesem Versuch keine Hinweise auf Hydratbildung.

### Beispiel 13

### Stabilität von WX-671 (freie Base)

Durch einwöchiges Rühren in Diisopropylether und Ethanol/Wasser (1:1) bei Raumtemperatur kristallisiert der Wirkstoff nicht bzw. zu sehr geringem Anteil in Modifikation A. Er wandelt sich in keine andere polymorphe Form um. Durch mechanischen Stress (Mörsern, Pressen mit 9 kbar) wandelt er sich ebenfalls nicht um, lediglich die Kristallinität nimmt weiter ab.

## Patentansprüche

1. Kristalline Modifikation von N-α-(2,4,6-Triisopropylphenylsulfonyl)-3-hydroxyamidino-(L)-phenylalanin-4-ethoxycarbonylpiperazid-Sulfat, **dadurch gekennzeichnet,**
**dass** sie im Wesentlichen die folgenden Peaks (Reflexe 2 Theta) gemäß einer Röntgendiffraktometrie-Analyse (Cu_{Kα} = 1,54178Å) aufweist:
3,7
10,3
12,1
13,8
16,4
19,2
22,2.

2. Kristalline Modifikation nach Anspruch 1, **dadurch gekennzeichnet, dass** sie N-α-(2,4,6-Triisopropylphenylsulfonyl)-3-hydroxyamidino-(L)-phenylalanin-4-ethoxycarbonylpiperazid-Sulfat im Einkristall umfasst.

3. Kristalline Modifikation von N-α-(2,4,6-Triisopropylphenylsulfonyl)-3-hydroxyamidino-(L)-phenylalanin-4-ethoxy-carbonylpiperazid als freie Base,
**dadurch gekennzeichnet,**
**dass** sie im Wesentlichen die folgenden Peaks (Reflexe 2 Theta) gemäß einer Röntgendiffraktometrie-Analyse (Cu_{Kα} = 1,54178Å) aufweist:
3,2
6,4
10,2
19,7.

4. Kristalline Modifikation nach Anspruch 3,
**dadurch gekennzeichnet,**
**dass** sie eine kristalline Modifikation von N-α-(2,4,6-Triisopropylphenylsulfonyl)-3-hydroxyamidino-(L)-phenylalanin-4-ethoxycarbonylpiperazid als freie Base im Einkristall umfasst.

5. Kristalline Modifikation nach einem der Ansprüche 1-2,
**dadurch gekennzeichnet,**
**dass** sie N-α-(2,4,6-Triisopropylphenylsulfonyl)-3-hydroxyamidino-(L)-phenylalanin-4-ethoxycarbonylpiperazid und Sulfat-Anionen in einem Molverhältnis von 1,5-2,5:1 aufweist.

6. Kristalline Modifikation nach Anspruch 5,
**dadurch gekennzeichnet,**
**dass** sie N-α-(2,4,6-Triisopropylphenylsulfonyl)-3-hydroxyamidino-(L)-phenylalanin-4-ethoxycarbonylpiperazid und Sulfat-Anionen in einem Molverhältnis von 2:1 aufweist.

7. Kristalline Modifikation nach einem der Ansprüche 1-2,
**dadurch gekennzeichnet,**
**dass** sie Einheiten aus jeweils etwa zwei Molekülen N-α-(2,4,6-Triisopropylphenylsulfonyl)-3-hydroxyamidino-(L)-phenylalanin-4-ethoxycarbonylpiperazid und einem Sulfat-Anion umfasst.

8. Kristalline Modifikation nach Anspruch 7,
**dadurch gekennzeichnet,**
**dass** die zwei Moleküle N-α-(2,4,6-Triisopropylphenylsulfonyl)-3-hydroxyamidino-(L)-phenylalanin-4-ethoxycarbonylpiperazid in einer Einheit in verschiedenen Konformationen vorliegen.

9. Kristalline Modifikation nach einem der vorhergehenden Ansprüche,
**dadurch gekennzeichnet,**
**dass** sie pro Mol Salz etwa drei Mol Wasser aufweist.

10. Verfahren zur Herstellung einer im wesentlichen kristallinen Modifikation von N-α-(2,4,6-Triisopropylphenylsulfonyl)-3-hydroxyamidino-(L)-phenylalanin-4-ethoxy-carbonylpiperazid nach einem der Ansprüche 1-9 umfassend die Schritte
(a) Bereitstellen der Verbindung N-α-(2,4,6-Triisopropylphenylsulfonyl)-3-hydroxyamidino-(L)-phenylalanin-4-ethoxycarbonylpiperazid oder eines ihrer Salze,
(b) Lösen und/oder Suspendieren der Verbindung oder des Salzes derselben aus Schritt (a) in einem zur Bildung der kristallinen Modifikation geeigneten Lösungsmittel,
(c) Abtrennen der kristallinen Modifikation.

11. Verfahren nach Anspruch 10, umfassend den zusätzlichen Schritt
(d) Umkristallisieren der kristallinen Modifikation aus Schritt (c) aus einem geeigneten Lösungsmittel oder einer Mischung von Lösungsmitteln.

12. Arzneimittel, enthaltend als Wirkstoff eine kristalline Modifikation nach einem der Ansprüche 1 bis 9, gegebenenfalls zusammen mit einem pharmazeutisch verträglichen Träger oder/und Hilfsstoff.

13. Arzneimittel nach Anspruch 12,
**dadurch gekennzeichnet,**
**dass** es ein oral, nasal, inhalativ, rektal oder/und parenteral verabreichbares Mittel ist.

14. Verwendung einer kristallinen Modifikation von N-α-(2,4,6-Triisopropylphenylsulfonyl)-3-hydroxyamidino-(L)-phenylalanin-4-ethoxycarbonylpiperazid nach einem der Ansprüche 1-9 zur Herstellung einer pharmazeutischen Zusammensetzung für die Bekämpfung von Krankheiten, die mit einer pathologischen Überexpression von Urokinase und/oder dem Urokinaserezeptor assoziiert sind.

15. Verwendung nach Anspruch 14 zur Tumorbehandlung und/oder - prävention.

16. Verwendung nach Anspruch 15 zur Behandlung oder Prävention der Metastasenbildung.

17. Verwendung nach Anspruch 15 zur Behandlung von Primärtumoren.

18. Verwendung nach einem der Ansprüche 14 bis 17, wobei eine oral verabreichbare Zusammensetzung hergestellt wird.

19. Verwendung nach Anspruch 18, wobei die Zusammensetzung in Form von Tabletten, Dragees, Kapseln, Pellets, Pulver, Zäpfchen, Lösungen, Sirup, Emulsionen, Liposomen oder/und Suspensionen hergestellt wird.

## Claims

1. Crystalline modification of N-α-(2,4,6-triisopropylphenylsulfonyl)-3-hydroxyamidino-(L)-phenylalanine-4-ethoxycarbonylpiperazide sulfate, **characterised in that** it substantially comprises the following peaks (reflexes 2 theta) according to an X-ray diffractometry analysis (Cu_{Kα} = 1.54178Å):
3.7
10.3
12.1
13.8
16.4
19.2
22.2.

2. Crystalline modification according to claim 1, **characterised in that** it has N-α-(2,4,6-triisopropylphenylsulfonyl)-3-hydroxyamidino-(L)-phenylalanine-4-ethoxycarbonylpiperazide sulfate in a single crystal.

3. Crystalline modification of N-α-(2,4,6-triisopropylphenylsulfonyl)-3-hydroxyamidino-(L)-phenylalanine-4-ethoxycarbonylpiperazide as a free base, **characterised in that** said modification essentially comprises the following peaks (reflexes 2 theta) according to an X-ray diffractometry analysis (Cu_{Kα} = 1.54178Å):
3.2
6.4
10.2
19.7.

4. Crystalline modification according to claim 3, **characterised in that** it has a crystalline modification of N-α-(2,4,6-triisopropylphenylsulfonyl)-3-hydroxyamidino-(L)-phenylalanine-4-ethoxycarbonylpiperazide as a free base in a single crystal.

5. Crystalline modification according to any of claims 1 to 2, **characterised in that** it comprises N-α-(2,4,6-triisopropylphenylsulfonyl)-3-hydroxyamidino-(L)-phenylalanine-4-ethoxycarbonylpiperazide and sulfate anions in a molar ratio of 1.5-2.5:1.

6. Crystalline modification according to claim 5, **characterised in that** it comprises N-α-(2,4,6-triisopropylphenylsulfonyl)-3-hydroxyamidino-(L)-phenylalanine-4-ethoxycarbonylpiperazide and sulfate anions in a molar ratio of 2:1.

7. Crystalline modification according to any of claims 1 to 2, **characterised in that** it has units of in each case approximately two molecules of N-α-(2,4,6-triisopropylphenylsulfonyl)-3-hydroxyamidino-(L)-phenylalanine-4-ethoxycarbonylpiperazide and a sulfate anion.

8. Crystalline modification according to claim 7, **characterised in that** the two molecules of N-α-(2,4,6-triisopropylphenylsulfonyl)-3-hydroxyamidino-(L)-phenylalanine-4-ethoxycarbonylpiperazide are present in one unit in various conformations.

9. Crystalline modification according to any of the preceding claims, **characterised in that** it contains approximately three moles of water per mole of salt.

10. Method for producing a substantially crystalline modification of N-α-(2,4,6-triisopropylphenylsulfonyl)-3-hydroxyamidino-(L)-phenylalanine-4-ethoxycarbonylpiperazide according to any of claims 1 to 9, having the steps of:
(a) preparing the compound N-α-(2,4,6-triisopropylphenylsulfonyl)-3-hydroxyamidino-(L)-phenylalanine-4-ethoxycarbonylpiperazide or one of its salts,
(b) dissolving and/or suspending the compound or the salt thereof from step (a) in a solvent suitable for the formation of the crystalline modification,
(c) separating the crystalline modification.

11. Method according to claim 10, having the additional step of
(d) recrystallising the crystalline modification from step (c) from a suitable solvent or a mixture of solvents.

12. Medicament containing as the active ingredient a crystalline modification according to any of claims 1 to 9, optionally together with a pharmaceutically tolerable carrier and/or excipient.

13. Medicament according to claim 12, **characterised in that** it is an orally, nasally, inhalatively, rectally and/or parenterally administrable agent.

14. Use of a crystalline modification of N-α-(2,4,6-triisopropylphenylsulfonyl)-3-hydroxyamidino-(L)-phenylalanine-4-ethoxycarbonylpiperazide according to any of claims 1 to 9 for producing a pharmaceutical composition for controlling diseases which are associated with a pathological overexpression of urokinase and/or the urokinase receptor.

15. Use according to claim 14 for tumour treatment and/or prevention.

16. Use according to claim 15 for the treatment or prevention of the formation of metastases.

17. Use according to claim 15 for the treatment of primary tumours.

18. Use according to any of claims 14 to 17, wherein an orally administrable composition is produced.

19. Use according to claim 18, wherein the composition is produced in the form of tablets, coated tablets, capsules, pellets, powder, suppositories, solutions, syrup, emulsions, liposomes or/and suspensions.

## Revendications

1. Modification cristalline du sulfate de N-α-(2,4,6-triisopropyl-phénylsulfonyl)-3-hydroxyamidino-(L)-phénylalanine-4-éthoxycarbonylpipérazide, **caractérisée**
**en ce qu'**elle comporte essentiellement les pics (réflexions 2 thêta) suivants selon une analyse par diffractométrie X (Cu_{Kα} = 1,54178Å):
3,7
10,3
12,1
13,8
16,4
19,2
22,2.

2. Modification cristalline selon la revendication 1, **caractérisée en ce qu'**elle comprend du sulfate de N-α-(2,4,6-triisopropylphénylsulfonyl)-3-hydroxyamidino-(L)-phénylalanine-4-éthoxycarbonylpipérazide dans un monocristal.

3. Modification cristalline du N-α-(2,4,6-triisopropylphénylsulfonyl)-3-hydroxyamidino-(L)-phénylalanine-4-éthoxycarbonylpipérazide sous forme de base libre,
**caractérisée**
**en ce qu'**elle comporte essentiellement les pics (réflexions 2 thêta) suivants selon une analyse par diffractométrie X (Cu_{Kα} = 1,54178Å):
3,2
6,4
10,2
19,7.

4. Modification cristalline selon la revendication 3,
**caractérisée**
**en ce qu'**elle comprend une modification cristalline du N-α-(2,4,6-triisopropylphénylsulfonyl)-3-hydroxyamidino-(L)-phénylalanine-4-éthoxycarbonylpipérazide sous forme de base libre dans un monocristal.

5. Modification cristalline selon l'une des revendications 1-2,
**caractérisée**
**en ce qu'**elle comporte du N-α-(2,4,6-triisopropylphénylsulfonyl)-3-hydroxy-amidino-(L)-phénylalanine-4-éthoxycarbonylpipérazide et des anions sulfate dans un rapport molaire de 1,5-2,5:1.

6. Modification cristalline selon la revendication 5,
**caractérisée**
**en ce qu'**elle comporte du N-α-(2,4,6-triisopropylphénylsulfonyl)-3-hydroxy-amidino-(L)-phénylalanine-4-éthoxycarbonylpipérazide et des anions sulfate dans un rapport molaire de 2:1.

7. Modification cristalline selon l'une des revendications 1-2,
**caractérisée**
**en ce qu'**elle comprend des unités à chaque fois d'environ deux molécules de N-α-(2,4,6-triisopropylphénylsulfonyl)-3-hydroxyamidino-(L)-phénylalanine-4-éthoxycarbonylpipérazide et d'un anion sulfate.

8. Modification cristalline selon la revendication 7,
**caractérisée**
**en ce que** les deux molécules de N-α-(2,4,6-triisopropylphénylsulfonyl)-3-hydroxyamidino-(L)-phénylalanine-4-éthoxycarbonylpipérazide sont présentes dans une unité dans des conformations différentes.

9. Modification cristalline selon l'une des revendications précédentes, **caractérisée**
**en ce qu'**elle comporte par mole de sel environ trois moles d'eau.

10. Procédé pour la préparation d'une modification essentiellement cristalline de N-α-(2,4,6-triisopropylphénylsulfonyl)-3-hydroxyamidino-(L)-phénylalanine-4-éthoxycarbonylpipérazide selon l'une des revendications 1-9 comprenant les étapes
(a) préparation du composé N-α-(2,4,6-triisopropylphénylsulfonyl)-3-hydroxyamidino-(L)-phénylalanine-4-éthoxycarbonylpipérazide ou de l'un de ses sels,
(b) dissolution et/ou mise en suspension du composé ou du sel de celui-ci provenant de l'étape (a) dans un solvant approprié pour la formation de la modification cristalline,
(c) séparation de la modification cristalline.

11. Procédé selon la revendication 10, comprenant l'étape supplémentaire
(d) recristallisation de la modification cristalline provenant de l'étape (c) dans un solvant approprié ou un mélange de solvants approprié.

12. Médicament contenant comme principe actif une modification cristalline selon l'une des revendications 1 à 9, éventuellement avec un vecteur et/ou auxiliaire pharmaceutiquement acceptable.

13. Médicament selon la revendication 12,
**caractérisé**
**en ce que** c'est un agent qui peut être administré par voie orale, nasale, par inhalation, par voie rectale et/ou parentérale.

14. Utilisation d'une modification cristalline du N-α-(2,4,6-triisopropylphénylsulfonyl)-3-hydroxyamidino-(L)-phénylalanine-4-éthoxycarbonylpipérazide selon l'une des revendications 1-9 pour la préparation d'une composition pharmaceutique pour la lutte contre les maladies qui sont associées avec une surexpression pathologique d'urokinase et/ou du récepteur d'urokinase.

15. Utilisation selon la revendication 14 pour le traitement et/ou la prévention des tumeurs.

16. Utilisation selon la revendication 15 pour le traitement ou la prévention de la formation de métastases.

17. Utilisation selon la revendication 15 pour le traitement des tumeurs primaires.

18. Utilisation selon l'une des revendications 14 à 17, où une composition qui peut être administrée par voie orale est préparée.

19. Utilisation selon la revendication 18, où la composition est préparée sous forme de comprimés, de dragées, de capsules, de pellets, de poudres, de suppositoires, de solutions, de sirops, d'émulsions, de liposomes et/ou de suspensions.
